# EUROPEAN PATENT APPLICATION

(11) **EP 3 366 315 A1**
(43) Date of publication of application: **29.08.2018**
(21) Application number: 18158071.3
(22) Date of filing: 22.02.2018
(51) Int. Cl.: A61L 2/28, C12Q 1/00

(54) **METHOD TO READ BIOLOGICAL INDICATOR**

(30) Priority: 23.02.2017 US 201715440360
(71) Applicant: Ethicon, Inc., Somerville, NJ 08876 (US)
(72) Inventor: FRYER, Benjamin M., Irvine, CA California 92618 (US)
(74) Representative: Carpmaels & Ransford LLP

(57) **Abstract**

A method for detecting biological activity in a biological indicator comprises the steps of providing a known quantity of spores containing a fixed number of copies of an enzyme and a liquid growth medium comprising substrates of the enzyme. The enzyme substrates have a first emission spectrum, and are configured to be converted by the enzyme to substrate derivatives having a second emission spectrum. The spores are exposed to the liquid growth medium, and the liquid growth medium is measured for the second emission spectrum, and either no change in the second emission spectrum or a linear increase in the second emission spectrum, is detected as a function of time.

## Description

### BACKGROUND

Re-usable medical devices such as certain surgical instruments, endoscopes, etc., may be sterilized before re-use in order to minimize the likelihood that a contaminated device might be used on a patient, which could cause an infection in the patient. Various sterilization techniques may be employed, such as steam, hydrogen peroxide, peracetic acid, and vapor phase sterilization, either with or without a gas plasma and ethylene oxide (EtO). Each of these methods may depend to a certain extent on the diffusion rates of the sterilization fluids (e.g., gases) upon or into the medical devices to be sterilized.

Before sterilization, medical devices may be packaged within containers or pouches having a semi-permeable barrier that allows transmission of the sterilizing fluid-sometimes referred to as a sterilant-but prevents admission of contaminating organisms, particularly post-sterilization and until the package is opened by medical personnel. For the sterilization cycle to be efficacious, the contaminating organisms within the package must be killed because any organisms that survive the sterilization cycle could multiply and re-contaminate the medical device. Diffusion of the sterilant may be particularly problematic for medical devices that have diffusion-restricted spaces therein because these diffusion-restricted spaces may reduce the likelihood that a sterilization cycle may be effective. For example, some endoscopes have one or more long narrow lumens into which the sterilant must diffuse in sufficient concentration for sufficient time to achieve a successful sterilization cycle.

Sterilization of medical devices may be performed with an automated sterilization system such as a STERRAD® System by Advanced Sterilization Products of Irvine, California. Examples of automated sterilization systems are described in U.S. Pat. No. 6,939,519, entitled "Power System for Sterilization Systems Employing Low Frequency Plasma," issued September 6, 2005, the disclosure of which is incorporated by reference herein; U.S. Pat. No. 6,852,279, entitled "Sterilization with Temperature-Controlled Diffusion Path," issued February 8, 2005, the disclosure of which is incorporated by reference herein; U.S. Pat. No. 6,852,277, entitled "Sterilization System Employing a Switching Module Adapter to Pulsate the Low Frequency Power Applied to a Plasma," issued February 8, 2005, the disclosure of which is incorporated by reference herein; U.S. Pat. No. 6,447,719, entitled "Power System for Sterilization Systems Employing Low Frequency Plasma," issued September 10, 2002, the disclosure of which is incorporated by reference herein; and U.S. Provisional Pat. App. No. 62/316,722, entitled "System and Method for Sterilizing Medical Devices," filed April 1, 2016, the disclosure of which is incorporated by reference herein.

Operator error may result in medical devices that are erroneously believed to be decontaminated being returned to service. Confirming that a sterilization cycle has been efficacious may help medical personnel avoid using a contaminated medical device on a patient. The sterilized medical device might not itself be checked for contaminating organisms because such an activity may introduce other contaminating organisms to the medical device, thereby re-contaminating it. Thus, an indirect check may be performed using a biological indicator. A biological indicator is a device that may be placed alongside or in proximity to a medical device being subject to a sterilization cycle, such that the biological indicator is subject to the same sterilization cycle as the medical device. For instance, a biological indictor having a predetermined quantity of microorganisms may be placed into a sterilization chamber alongside a medical device and subject to a sterilization cycle. After the cycle is complete, the microorganisms in the biological indicator may be cultured to determine whether any of the microorganisms survived the cycle. The presence or absence of living microorganisms in the biological indicator will indicate whether the sterilization cycle was effective.

While a variety of systems and methods have been made and used for medical device sterilization, it is believed that no one prior to the inventor(s) has made or used the technology as described herein.

### BRIEF DESCRIPTION OF THE DRAWINGS

It is believed the present invention will be better understood from the following description of certain examples taken in conjunction with the accompanying drawings, in which like reference numerals identify the same elements and in which:
FIG. 1 depicts a schematic view of an exemplary medical device sterilizing cabinet;
FIG. 2 depicts a flowchart of an exemplary set of steps that may be carried out as part of a sterilization cycle performed by the sterilizing cabinet of FIG. 1;
FIG. 3 depicts a schematic view of an exemplary biological indicator assembly that may be placed with a medical device in the sterilizing cabinet of FIG. 1;
FIG. 4 depicts a schematic view of an exemplary indicator analyzer that may be used to process the biological indicator assembly of FIG. 3 after the biological indicator assembly has undergone a sterilization cycle in the sterilizing cabinet of FIG. 1;
FIG. 5 depicts a flowchart showing an exemplary set of steps that may be performed by a user of the indicator analyzer of FIG. 4 in preparation for analysis of the biological indicator assembly of FIG. 3; and
FIG. 6 depicts a flowchart of an exemplary method for detecting efficacy of a sterilization cycle using a version of the biological indicator assembly of FIG. 3.

### DETAILED DESCRIPTION

The following description of certain examples of the technology should not be used to limit its scope. Other examples, features, aspects, embodiments, and advantages of the technology will become apparent to those skilled in the art from the following description, which is by way of illustration, one of the best modes contemplated for carrying out the technology. As will be realized, the technology described herein is capable of other different and obvious aspects, all without departing from the technology. Accordingly, the drawings and descriptions should be regarded as illustrative in nature and not restrictive.

It is further understood that any one or more of the teachings, expressions, embodiments, examples, etc. described herein may be combined with any one or more of the other teachings, expressions, embodiments, examples, etc. that are described herein. The following-described teachings, expressions, embodiments, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

### I. Exemplary Sterilizing Cabinet

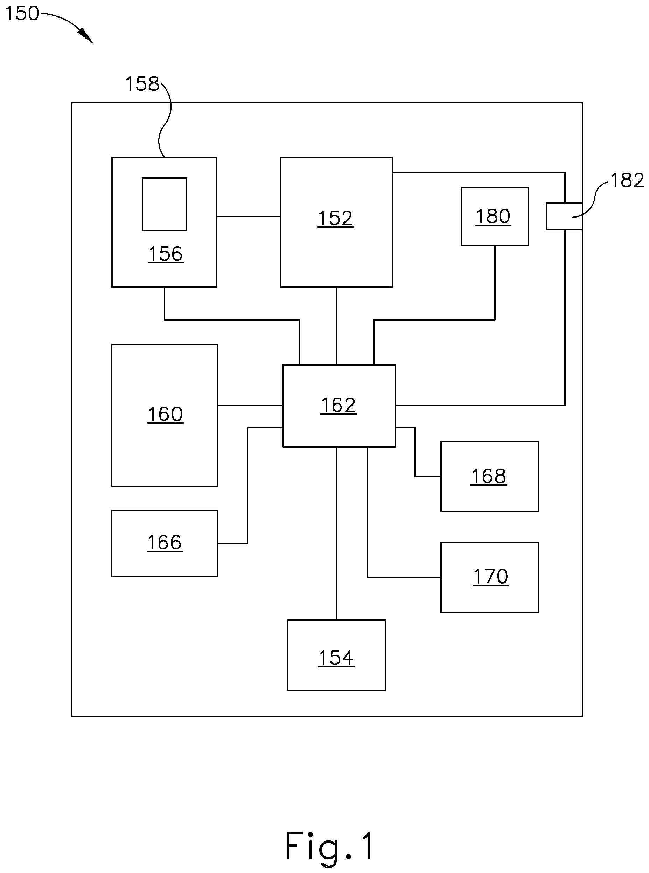

FIG. 1 depicts an exemplary sterilizing cabinet (150) that is operable to sterilize medical devices such as endoscopes, etc. Sterilizing cabinet (150) of the present example includes a sterilization chamber (152), which is configured to receive one or more medical devices for sterilization. While not shown, sterilizing cabinet (150) also includes a door that opens and closes sterilization chamber (152) in response to actuation of a kick plate. An operator may thereby open and close sterilization chamber (152) in a hands-free fashion. Of course, any other suitable features may be used to provide selective access to sterilization chamber. Sterilizing cabinet (150) also includes a sterilization module (156) that is operable to dispense a sterilant into sterilization chamber (152) in order to sterilize medical devices contained in sterilization chamber (152). In the present example, sterilization module (156) is configured to receive replaceable sterilant cartridges (158) containing a certain amount of sterilant. By way of example only, each sterilant cartridge (158) may contain enough sterilant to perform five sterilization procedures.

In the present example, sterilization module (156) is operable to apply a sterilant in the form of a vapor within sterilization chamber (152). By way of example only, sterilization module (156) may comprise a combination of a vaporizer and a condenser. The vaporizer may include a chamber that receives a particular concentration of sterilant solution (e.g., a liquid hydrogen peroxide solution with a concentration of approximately 59% nominal, or between approximately 58% and approximately 59.6%); where the sterilant solution changes phase from liquid to vapor. The condenser may optionally provide condensation of the sterilant solution vapor, and the concentration of the sterilant solution may be thereby increased (e.g., from approximately 59% nominal to somewhere between approximately 83% nominal and approximately 95% nominal), by removal of water vapor. Alternatively, any other suitable methods and components may be used to apply sterilant in the form of a vapor within sterilization chamber (152). In any case, to supplement the application of the sterilant in the form of a vapor, the sterilant may also be applied (in liquid form) to the inside of lumen(s) and/or other internal spaces within the medical device and/or the outside of the medical device, before the medical device is placed in sterilization chamber (152). In such versions, the sterilant may evaporate while a vacuum is applied to sterilization chamber (152) and even after vacuum is applied; and provide more concentration of sterilant to the areas of the medical device with less penetration range, thereby further promoting effective sterilization.

Sterilizing cabinet (150) of the present example further includes a touch screen display (160). Touch screen display (160) is operable to render the various user interface display screens, such as those described in U.S. Provisional Pat. App. No. 62/316,722, the disclosure of which is incorporated by reference herein. Of course, touch screen display (160) may display various other screens as well. Touch screen display (160) is further configured to receive user input in the form of the user contacting touch screen display (160) in accordance with conventional touch screen technology. In addition, or in the alternative, sterilizing cabinet (150) may include various other kinds of user input features, including but not limited to buttons, keypads, keyboards, a mouse, a trackball, etc.

Sterilizing cabinet (150) of the present example further includes a processor (162), which is in communication with sterilization module (156) and with touch screen display (160). Processor (162) is operable to execute control algorithms to drive sterilization module (156) in accordance with user input. Processor (162) is further operable to execute instructions to display the various screens on touch screen display (160); and to process instructions received from a user via touch screen display (160) (and/or via other user input features). Processor (162) is also in communication with various other components of sterilization cabinet (150) and is thereby operable to drive those components and/or process input and/or other data from those components. Various suitable components and configurations that may be used to form processor (162) will be apparent to those of ordinary skill in the art in view of the teachings herein.

Sterilizing cabinet (150) of the present example further includes a communication module (154). Communication module (154) is configured to enable bidirectional communication between sterilizing cabinet (150) and a communication hub (not shown), a server, and/or other equipment. In some versions, communication module (154) is configured to communicate with a hub in accordance with at least some of the teachings of U.S. Patent App. No. 62/376,517, entitled "Apparatus and Method to Link Medical Device Sterilization Equipment," filed August 18, 2016, the disclosure of which is incorporated by reference herein. By way of example only, communication module (154) may be configured to provide wired and/or wireless communication via as Ethernet, Wi-Fi, Bluetooth, USB, infrared, NFC, and/or other technologies. Various suitable components and configurations that may be used to form communication module (154) will be apparent to those of ordinary skill in the art in view of the teachings herein. Communications that are sent from or received through communication module (154) are processed through processor (162).

Sterilizing cabinet (150) of the present example further includes an identification tag reader (166), which is operable to read an identification tag of a biological indicator as described herein. By way of example only, identification tag reader (166) may comprise an optical reader that is operable to read an optical identification tag (e.g., barcode, data matrix code, QR code, etc.) of a biological indicator. In addition, or in the alternative, identification tag reader (166) may comprise an RFID reader that is operable to read an RFID identification tag of a biological indicator. In some versions where identification tag reader (166) comprises an RFID reader, identification tag reader (166) is also operable to write information to an RFID tag of a biological indicator. Various suitable components and configurations that may be used to form identification tag reader (166) will be apparent to those of ordinary skill in the art in view of the teachings herein. Data received through identification tag reader (166) is processed through processor (162).

Sterilizing cabinet (150) of the present example further includes a memory (168), which is operable to store control logic and instructions and that are executed by processor (162) to drive components such as sterilization module (156), touch screen display (160), communication module (154), and identification tag reader (166). Memory (168) may also be used to store results associated with setup of a sterilization cycle, performance of a load conditioning cycle, performance of a sterilization cycle, and/or various other kinds of information. Various suitable forms that memory (168) may take, as well as various ways in which memory (168) may be used, will be apparent to those of ordinary skill in the art in view of the teachings herein.

Sterilizing cabinet (150) of the present example further includes a printer (170), which is operable to print information such as results associated with setup of a sterilization cycle, performance of a load conditioning cycle, performance of a sterilization cycle, and/or various other kinds of information. By way of example only, printer (170) may comprise a thermal printer, though of course any other suitable kind of printer may be used. Various suitable forms that printer (170) may take, as well as various ways in which printer (170) may be used, will be apparent to those of ordinary skill in the art in view of the teachings herein. It should also be understood that printer (170) is merely optional and may be omitted in some versions.

Sterilizing cabinet (150) of the present example further includes a vacuum source (180) and a venting valve (182). Vacuum source (180) is in fluid communication with sterilization chamber (152) and is also in communication with processor (162). Thus, processor (162) is operable to selectively activate vacuum source (180) in accordance with one or more control algorithms. When vacuum source (180) is activated, vacuum source (180) is operable to reduce the pressure within sterilization chamber (152) as will be described in greater detail below. Venting valve (182) is also in fluid communication with sterilization chamber (152). In addition, venting valve (182) is in communication with processor (162) such that processor (162) is operable to selectively activate venting valve (182) in accordance with one or more control algorithms. When venting valve (182) is activated, venting valve (182) is operable to vent sterilization chamber (152) to atmosphere as will be described in greater detail below. Various suitable components that may be used to provide vacuum source (180) and venting valve (182) will be apparent to those of ordinary skill in the art in view of the teachings herein.

In addition to the foregoing, sterilizing cabinet (150) may be configured and operable in accordance with at least some of the teachings of U.S. Pat. No. 6,939,519, the disclosure of which is incorporated by reference herein; U.S. Pat. No. 6,852,279, the disclosure of which is incorporated by reference herein; U.S. Pat. No. 6,852,277, the disclosure of which is incorporated by reference herein; U.S. Pat. No. 6,447,719, the disclosure of which is incorporated by reference herein; U.S. Pat. No. 6,365,102, the disclosure of which is incorporated by reference herein; U.S. Pat. No. 6,325,972, the disclosure of which is incorporated by reference herein; and/or U.S. Provisional Patent App. No. 62/316,722, the disclosure of which is incorporated by reference herein.

### II. Exemplary Sterilization Process

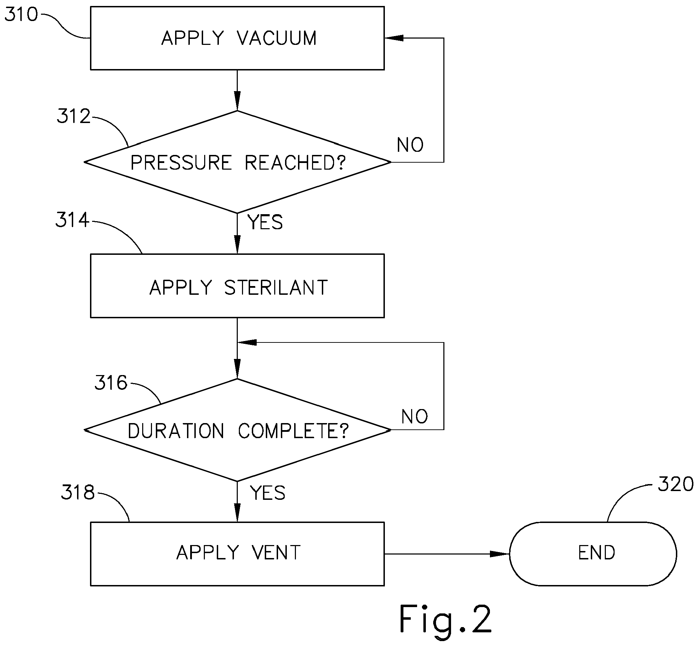

FIG. 2 depicts exemplary steps that may be performed by sterilizing cabinet (150) to provide a sterilization cycle on a used medical device, such as an endoscope. In particular, the cycle may begin with a vacuum being applied (block 310) within sterilization chamber (152). In order to provide such a vacuum, processor (162) may activate vacuum source (180) in accordance with a control algorithm. Processor (162) will then determine (block 312) whether a sufficient pressure level has been reached within sterilization chamber (152). By way of example only, processor (162) may monitor data from one or more pressure sensors within sterilization chamber (152) as part of the determination step (block 312). Alternatively, processor (162) may simply activate vacuum source (180) for a predetermined time period and assume that the appropriate pressure has been reached in sterilization (152) based upon the duration for which vacuum source (180) is activated. Other suitable ways in which processor (162) may determine (block 312) whether a sufficient pressure level has been reached within sterilization chamber (152) will be apparent to those of ordinary skill in the art in view of the teachings herein. Until the appropriate pressure level has been reached within sterilization chamber (152), vacuum source (180) will remain activated.

Once sterilization chamber (152) reaches an appropriate pressure level (e.g., between approximately 0.2 torr and approximately 10 torr), processor (162) then activates sterilization module (156) to apply a sterilant (block 314) in sterilization chamber (152). This stage of the process may be referred to as the "transfer phase." By way of example only, the sterilant may comprise a vapor of oxidizing agent such as hydrogen peroxide, peroxy acids (e.g. peracetic acid, performic acid, etc.), ozone, or a mixture thereof. Furthermore, the sterilant may comprise chlorine dioxide. Various other suitable forms that the sterilant may take are described herein; while other forms will be apparent to those of ordinary skill in the art in view of the teachings herein. Once the sterilant has been applied (block 314) to sterilization chamber (152), processor (162) monitors the time (block 316) to determine whether a sufficient, predetermined duration has passed. By way of example only, this predetermined duration may be anywhere from a few seconds to several minutes. Until the predetermined duration has passed, sterilization chamber (152) remains in a sealed state at the above-noted predetermined pressure level, with the applied sterilant acting upon the medical device(s) contained within sterilization chamber (152).

After the predetermined duration has passed, processor (162) activates (block 318) venting valve (182) to vent sterilization chamber (152) to atmosphere. In some versions, sterilization chamber (152) is allowed to reach atmospheric pressure, while in other versions sterilization chamber (152) only reaches sub-atmospheric pressure. The venting stage of the process may be referred to as the "diffusion phase." In the present example, the sterilization cycle is then complete (block 320) after completion of the diffusion phase. In some other instances, vacuum is again applied to sterilization chamber (152) after completion of the diffusion phase; and then a plasma is applied to sterilization chamber (152), It should be understood that the entire sterilization cycle shown in FIG. 2 (including the above-noted variation where a subsequent vacuum then sterilization are applied) may be repeated one or more times after being completed once. In other words, a medical device may remain within sterilization chamber (152) and experience two or more iterations of the entire cycle shown in FIG. 2 (including the above-noted variation where a subsequent vacuum then sterilization are applied).

In some variations, a pre-plasma may be applied in the sterilization cycle to heat up the medical device contained in sterilization chamber (152). By way of example only, plasma may be applied between applying a vacuum (block 310) and applying sterilant (block 314). In addition, or in the alternative, a post-plasma may be applied at the end of the sterilization cycle to degrade any residual sterilant that may be adsorbed to the surface of the medical device contained in sterilization chamber (152). It should be understood that before applying the post-plasma, a vacuum would first need to be applied to sterilization chamber (152).

By way of example only, the process depicted in FIG. 2 may be carried out at temperatures where the walls of sterilization chamber (152) are between approximately 30°C and approximately 56°C, or more particularly between approximately 47°C and approximately 56°C, or even more particularly approximately 50°C; and where the temperature of the medical device in sterilization chamber (152) is between approximately 5-10°C and approximately 40-55°C.

In addition to the foregoing, sterilizing cabinet (150) may be configured to perform sterilization processes in accordance with at least some of the teachings of U.S. Pat. No. 6,939,519, the disclosure of which is incorporated by reference herein; U.S. Pat. No. 6,852,279, the disclosure of which is incorporated by reference herein; U.S. Pat. No. 6,852,277, the disclosure of which is incorporated by reference herein; U.S. Pat. No. 6,447,719, the disclosure of which is incorporated by reference herein; U.S. Pat. No. 6,365,102, the disclosure of which is incorporated by reference herein; U.S. Pat. No. 6,325,972, the disclosure of which is incorporated by reference herein; and/or U.S. Provisional Patent App. No. 62/316,722, the disclosure of which is incorporated by reference herein.

While the foregoing examples are described in the context of sterilizing medical devices, and particularly endoscopes, it should be understood that the teachings herein may also be readily applied in the context of sterilizing various other kinds of articles. The teachings are not limited to endoscopes or other medical devices. Other suitable articles that may be sterilized in accordance with the teachings herein will be apparent to those of ordinary skill in the art.

### III. Exemplary Biological Indicator Assembly

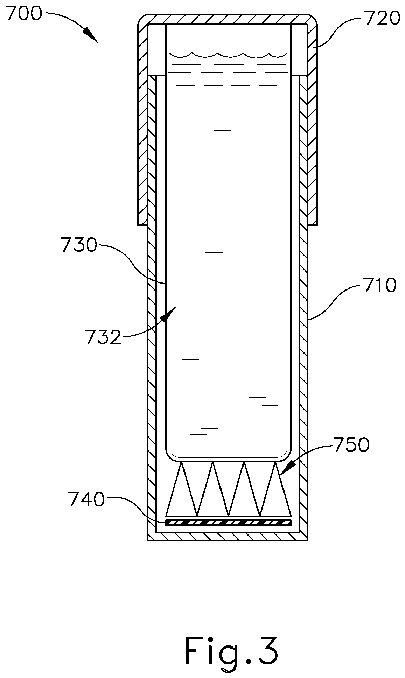

In some instances, it may be desirable to include a biological indicator along with the medical device in sterilization chamber (152) during the sterilization process in order to ensure that the sterilization process was successful. FIG. 3 shows an exemplary form that such a biological indicator may take. In particular, FIG. 3 shows a biological indicator (700) that includes a housing (710), a cap (720), an ampoule (730), and a carrier (740). Housing (710) is formed of a transparent material (e.g., clear plastic, glass, etc.) and is hollow such that housing (710) insertably receives ampoule (730). Ampoule (730) is also formed of a transparent material (e.g., clear plastic, glass, etc.) and contains a fluid (732). By way of example only, fluid (732) may comprise a liquid growth medium. Such a liquid growth medium is capable of, with incubation, promoting growth of any viable microorganisms it contacts. Fluid (732) also includes a fluorophore whose fluorescence depends on the number of living microorganisms that may be contained in the liquid medium of fluid (732) after ampoule (730) is fractured as will be described in greater detail below. Fluid (732) is sealed within ampoule (730).

Carrier (740) provides a source of biological activity that is resistant to a particular sterilization process. The phrase "biological activity" generally refers to any specific catalytic process or groups of processes associated with a biological cell. Non-limiting examples of biological activities include catabolic enzyme activities (e.g., carbohydrate fermentation pathways), anabolic enzyme activities (e.g., nucleic acid, amino acid, or protein synthesis), coupled reactions (e.g., a metabolic pathway), biomolecule-mediated redox reactions (e.g., electron transport systems) and bioluminescent reactions. Exemplary biological indicators (700) include a viable quantity, or culture, of one or more known sources of biological activity (e.g., species of microorganism). Such sources of biological activity can be in the form of microbial spores.

The source of biological activity in biological indicator (700) either is killed by a successful sterilization cycle or survives if the sterilization cycle is not effective for some reason. Bacterial spores, rather than the vegetative form of the organisms, may be used at least in part, because vegetative bacteria may be relatively easily killed by sterilizing processes. Spores can also have superior storage characteristics and can remain in their dormant state for years. As a result, in some exemplary biological indicators (700), sterilization of an inoculum of a standardized spore strain can provide a high degree of confidence that inactivation of all microorganisms in a sterilizing chamber has occurred.

The present disclosure describes the one or more sources of biological activity used in the biological indicator as being "microorganisms" or "spores." However, it should be understood that the type of source (e.g., spore) used in a particular embodiment of the biological indicator (700) is selected for being highly resistant to the particular sterilization process contemplated. Accordingly, different exemplary biological indicators (700) of the present disclosure may use different sources of biological activity, depending on the sterilization process for which the particular biological indicators (700) are intended. The term "spores" is used throughout the present disclosure for simplicity, but it should be understood that any suitable sources of biological activity, such those selected from: microorganisms (e.g., bacteria, fungi, viruses, etc.), spores (e.g., bacterial, fungal, etc.), enzymes, substrates for enzymatic activity, ATP, microbial metabolites, or combinations thereof, may instead be used in exemplary biological indicators (700).

By way of example only, carrier (740) may support, for example, or be impregnated with, spores from Bacillus, Geobacillus, and Clostridium species. Carrier (740) may be water-absorbent and may be formed of filter material. Sheet-like materials such as cloth, paper, nonwoven polypropylene, rayon or nylon, and microporous polymeric materials may also be used to form carrier (740). Non-water absorbent materials may also be used to form carrier (740), such as metals (e.g., aluminum or stainless steel), glass (e.g., glass beads or glass fibers), porcelain, or plastic. Of course, combinations of the foregoing materials may also be used to form carrier (740).

Ampoule (730) is configured as a frangible component of biological indicator (700), such that ampoule (730) may be fractured within housing to release fluid (732) in housing (710). To assist in the fracture of ampoule (730), a set of fracturing features (750) are disposed in the bottom of housing (710). While fracturing features (750) are shown as spikes in FIG. 3, it should be understood that this is merely illustrative. Fracturing features (750) may take any other suitable form. To further assist in the fracture of ampoule (730), cap (720) is configured to slide downwardly along housing (710) to press ampoule (730) against fracturing features (750). This may be done right before biological indicator (700) is inserted into indicator analyzer (800) as described in greater detail below. It should be understood that ampoule (730) would remain intact while biological indicator (700) is in sterilizing cabinet (150) during a sterilization process. Cap (720) may include one or more openings that allow gasses (e.g., air or sterilant, etc.) to pass into housing (710) before cap (720) is pressed downwardly relative to housing (710) to fracture ampoule (730). These openings may thus enable the microorganisms on carrier (740) to be destroyed by the sterilization process (block 208). However, after cap (720) is pressed downwardly relative to housing (710) to fracture ampoule (730), these one or more openings may be sealed to contain the released fluid (732) in housing (710). When fluid (732) is released from ampoule (730), the released fluid eventually reaches carrier (740), thereby initiating an incubation process with any living microorganisms remaining on carrier (740).

While not shown in FIG. 3, housing (710) may also include an identification tag. Such an identification tag may include a machine readable feature that is capable of being read by identification tag reader (166) of sterilizing cabinet (150) and indicator analyzer (800). By way of example only, the identification tag may comprise an optical code (e.g., a barcode, a data matrix code, a QR code, etc.), an RFID tag, and/or any other suitable kind of machine readable identifier. In addition, the identification tag may include human readable features such as text, numbers, color coding, etc.

Cap (720) may also include a color changing feature. Such a color changing feature may serve as a chemical indicator that changes color when biological indicator (700) is exposed to the sterilant of sterilizing cabinet (150). In some versions, the color changing feature simply changes between two distinctive colors, with one of the colors indicating no exposure to a sterilant and the other color indicating at least some exposure to a sterilant. In some other versions, the color changing feature changes along a range of colors based on the extent to which biological indicator (700) has been exposed to a sterilant. In other words, the color change may be proportional to the degree of sterilant exposure. As yet another merely illustrative example, a color changing feature may change color (e.g., from one of two available colors to another of two available colors; or along a range of colors) in response to a combination of two or more parameters. For instance, such parameters may include (but need not be limited to) time and concentration of sterilant; time, temperature, and concentration or amount/dose of sterilant received; and/or other parameters as will be apparent to those of ordinary skill in the art in view of the teachings herein.

In addition to or in lieu of the foregoing, biological indicator (700) may be configured and operable in accordance with at least some of the teachings of U.S. Pat. App. No. 15/057,768, entitled "Self-Contained Biological Indicator," filed March 1, 2016, the disclosure of which is incorporated by reference herein. Other suitable forms that biological indicator (700) may take will be apparent to those of ordinary skill in the art in view of the teachings herein

### IV. Exemplary Biological Indicator Analyzer

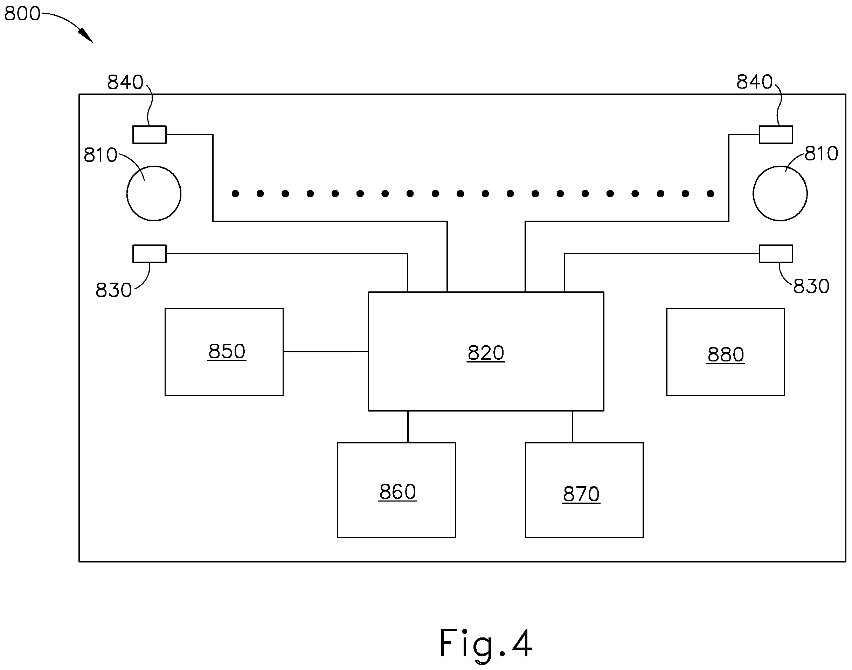

FIG. 4 depicts an exemplary biological indicator analyzer (800) that is operable to analyze a biological indicator (700) that has been subject to a sterilization cycle in sterilization cabinet (150). Biological indicator analyzer (800) is thus capable of determining whether the sterilization cycle was effective. Biological indicator analyzer (800) of this example comprises a plurality of wells (810), each of which is configured to insertingly receive a respective biological indicator (700). While two wells (810) are shown, it should be understood that any other suitable number of wells (810) may be provided, including eight wells (810), less than eight wells (810), or more than eight wells (810). Biological indicator analyzer (800) also includes a processor (820) that is operable to execute instructions and control algorithms, process information, etc.

Each well (810) has an associated light source (830) and sensor (840). Each light source (830) is configured to project light through housing (710) of the biological indicator (700) that is inserted in the corresponding well (810); and each sensor (840) is operable to detect light fluoresced by fluid (732) contained in housing (710). By way of example only, light source (830) may be in the form of a laser that is configured to emit ultraviolet light. Various other suitable forms that light source (830) may take will be apparent to those of ordinary skill in the art in view of the teachings herein. By way of further example only, sensor (840) may comprise a charge coupled device (CCD). Various other suitable forms that sensor (840) may take will be apparent to those of ordinary skill in the art in view of the teachings herein. The fluorescence of fluid (732) will depend on the number of living microorganisms contained in the medium of fluid (732). Thus, sensor (840) is configured to detect the presence of living microorganisms in fluid (732) based on the degree to which fluid (732) fluoresces in response to light from light source (830).

Biological indicator analyzer (800) of the present example further includes a touch screen display (850). Touch screen display (850) is operable to render various user interface display screens associated with operation of biological indicator analyzer (800). Touch screen display (850) is further configured to receive user input in the form of the user contacting touch screen display (850) in accordance with conventional touch screen technology. In addition, or in the alternative, biological indicator analyzer (800) may include various other kinds of user input features, including but not limited to buttons, keypads, keyboards, a mouse, a trackball, etc. Displays provided through touch screen display (850) may be driven by processor (820). User inputs received through touch screen display (850) may be processed by processor (820).

Biological indicator analyzer (800) of the present example further includes a communication module (860). Communication module (860) is configured to enable bidirectional communication between biological indicator analyzer (800) and a communication hub (not shown), a server, and/or other equipment. In some versions, communication module (860) is configured to communicate with a hub in accordance with at least some of the teachings of U.S. Patent App. No. 62/376,517, entitled "Apparatus and Method to Link Medical Device Sterilization Equipment," filed August 18, 2016, the disclosure of which is incorporated by reference herein. By way of example only, communication module (860) may be configured to provide wired and/or wireless communication via as Ethernet, Wi-Fi, Bluetooth, USB, infrared, NFC, and/or other technologies. Various suitable components and configurations that may be used to form communication module (860) will be apparent to those of ordinary skill in the art in view of the teachings herein. Communications that are sent from or received through communication module (860) are processed through processor (820).

Biological indicator analyzer (800) of the present example further includes an identification tag reader (870), which is operable to read an identification tag of biological indicator (700) as described herein. It should be understood that identification tag reader (870) may be used to identify biological indicator (700) before biological indicator (700) is analyzed. By way of example only, identification tag reader (870) may comprise an optical reader that is operable to read an optical identification tag (e.g., barcode, data matrix code, QR code, etc.) of a biological indicator (700). In addition, or in the alternative, identification tag reader (870) may comprise an RFID reader that is operable to read an RFID identification tag of a biological indicator (700). In some versions where identification tag reader (870) comprises an RFID reader, identification tag reader (870) is also operable to write information to an RFID tag of a biological indicator (700). Various suitable components and configurations that may be used to form identification tag reader (870) will be apparent to those of ordinary skill in the art in view of the teachings herein. Data received through identification tag reader (870) is processed through processor (820).

Biological indicator analyzer (800) of the present example further includes a memory (880), which is operable to store control logic and instructions and that are executed by processor (820) to drive components such as light source (830), touch screen display (850), communication module (860), and identification tag reader (870). Memory (880) may also be used to store results associated with performance of biological indicator analysis, and/or various other kinds of information. Various suitable forms that memory (880) may take, as well as various ways in which memory (880) may be used, will be apparent to those of ordinary skill in the art in view of the teachings herein.

### V. Exemplary Method of Using Indicator Analyzer to Detect Efficacy of a Sterilization Cycle

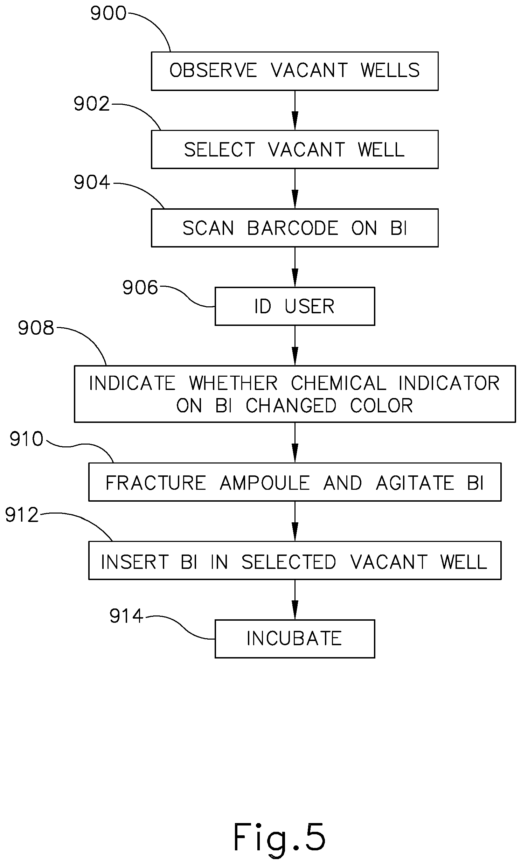

FIG. 5 shows an exemplary set of steps that may be used to initiate biological indicator (700) analysis cycle by biological indicator analyzer (800). As a first step, the user may observe which wells (810) are vacant (block 900) and select a vacant well (block 902). In some versions, touch screen display (850) presents a number next to each vacant well (810), such that the operator simply touches the number associated with the selected vacant well (810) in order to effect selection of that vacant well (block 902). Next, a display screen on touch screen display (850) may prompt the user to place the identification tag of biological indicator (700) near identification tag reader (870) to enable identification tag reader (870) to read the identification tag of biological indicator (700). As part of this prompting, touch screen display (850) may point to the location of identification tag reader (870) to assist the user in finding identification tag reader (870). The user may then use identification tag reader (870) to read the identification tag of biological indicator (700) (block 904).

A display screen on touch screen display (850) may then prompt the user to identify himself or herself. The user may then manipulate touch screen display (850) to identify himself or herself (block 906). A display screen on touch screen display (850) may then prompt the user to indicate whether the chemical indicator on cap (720) of biological indicator (700) has changed color. The user may then manipulate touch screen display (850) to indicate whether the chemical indicator on cap (720) of biological indicator (700) has changed color (block 908).

A display screen on touch screen display (850) may then prompt the user to prepare biological indicator (700) for loading into the selected well (810) by fracturing ampoule (730) and agitating biological indicator (700). The operator may then fracture ampoule (730) by pressing on cap (720), then agitate biological indicator (700) (block 910) to ensure proper mixing of fluid (732) with carrier (740). The user may then quickly place biological indicator (700) in the selected well (810) (block 912). In some instances, it may be desirable to insert biological indicator (700) in the selected well (810) (block 912) immediately after fracturing ampoule (730) and agitating biological indicator (700) (block 910).

In some versions, indicator analyzer (800) is configured to determine whether the user appropriately completed the step of fracturing ampoule (730) and agitating biological indicator (700) (block 910) before inserting biological indicator (700) in the selected well (810) (block 912). By way of example only, this may be determined based on how sensor (840) detects light emitted by light source (830) after biological indicator (700) is inserted in the selected well (810). In the event that indicator analyzer (800) determines that the user failed to appropriately complete the step of fracturing ampoule (730) and agitating biological indicator (700) (block 910) before inserting biological indicator (700) in the selected well (810) (block 912), touch screen display (850) may prompt the user to withdraw biological indicator (700) from well (810) and properly complete the step of fracturing ampoule (730) and agitating biological indicator (700) (block 910).

To the extent that the user has properly completed the step of fracturing ampoule (730) and agitating biological indicator (700) (block 910), and then inserted biological indicator (700) in the selected well (block 912), biological indicator (700) is allowed to sit in well (810) for an incubation period (block 914). During the incubation period (block 914), light source (830) associated with the selected well (810) is activated and sensor (840) monitors responsive fluorescence of fluid (732) in indicator (700). Well (810) may also be heated (e.g., to approximately 60°C) during the incubation period (block 914). As noted above, fluid (732) includes a fluorophore whose fluorescence depends on the number of microorganisms contained in the medium. Thus, sensor (840) can detect the presence of living microorganisms (from carrier (740)) in fluid (732) based on the fluorescence of fluid (732). It should therefore be understood that after a suitable incubation period has passed, indicator analyzer (800) will conclude whether any of the microorganisms that were on carrier (740) (i.e., before the sterilization cycle in sterilization cabinet (150)) survived the sterilization cycle in sterilization cabinet (150), based on the fluorescence of fluid (732) as sensed by sensor (840).

By way of example only, the incubation period (block 914) may be approximately 30 minutes. Alternatively, the incubation period may be substantially longer (e.g., one or more hours), shorter, or of any other suitable duration. During the incubation period (block 914), touch screen display (850) may provide a graphical representation of the amount of time remaining in the incubation period. When more than one well (810) is occupied by a corresponding biological indicator (700), touch screen display (850) may provide a graphical representation of the amount of time remaining in the incubation period for each occupied well (810).

After the incubation period (block 914) is complete, biological indicator analyzer (800) can determine whether any of the microorganisms that were on carrier (740) (i.e., before the sterilization cycle in sterilization cabinet (150)) survived the sterilization cycle in sterilization cabinet (150), based on the fluorescence of fluid (732) as sensed by sensor (840). Thus, biological indicator analyzer (800) can determine whether biological indicator (700) passes or fails analysis. In this sense, a "pass" result indicates that no living microorganisms are present in biological indicator (700), which indicates that the sterilization cycle in sterilization cabinet (150) was successful. A "fail" result indicates that living microorganisms are present in biological indicator (700), which indicates that the sterilization cycle in sterilization cabinet (150) was unsuccessful.

In the event of a "pass" result, touch screen display (850) may present a screen to the user indicating that biological indicator (700) passed the analysis. Touch screen display (850) may also prompt the user to remove biological indicator (700) from well (810) and appropriately discard the used biological indicator (700). In the event of a "fail" result, touch screen display (850) may present a screen to the user indicating that biological indicator (700) failed the analysis. Touch screen display (850) may then prompt the user to identify himself or herself. The user may then manipulate touch screen display (850) to identify himself or herself. Touch screen display (850) may then prompt the user to remove biological indicator (700) from well (810) and appropriately discard the used biological indicator (700). In either or both cases where there is a "pass" result or a "fail" result, communication module (860) may transmit the results to a communication hub, server, and/or other device(s).

In addition to the foregoing, indicator analyzer (800) may be configured and operable in accordance with at least some of the teachings of U.S. Patent App. No. 62/316,722, entitled "System and Method for Sterilizing Medical Devices," filed April 1, 2016, the disclosure of which is incorporated by reference herein.

### VI. Exemplary Alternative Methods for Detecting Efficacy of a Sterilization Cycle

As noted above, the efficacy of a sterilization cycle may be determined by subjecting a biological indicator (700) alongside, or in proximity to, a medical device being subject to the same sterilization cycle in sterilizing cabinet (150). For example, a biological indicator (700) having a predetermined quantity of microorganisms may be placed into a sterilization chamber (152) alongside a medical device and subject to the sterilization cycle. After the cycle is complete, the microorganisms in the biological indicator (700) may be analyzed using indicator analyzer (800) to determine whether any of the microorganisms survived the cycle. Alternatively, the microorganisms in the biological indicator (700) may be cultured to determine whether any of the microorganisms survived the cycle. The presence or absence of living microorganisms in the biological indicator (700) will be deemed indicative of whether the sterilization cycle effectively sterilized the medical device.

Presence or absence of living microorganisms in a biological indicator (700) may be determined by including an indicator dye, such as a pH indicator dye or a redox dye, in the liquid growth medium (732) that is used to incubate the microorganisms after they have been subjected to a sterilization cycle. After a sufficient incubation period, the biological indicator (700) is observed for signs of microorganism metabolism and/or growth that would indicate that the sterilization cycle was insufficient to destroy all of the sources of biological activity in the biological indicator (700) and likewise, the medical device. If a sterilization cycle is found to be insufficient, the medical device is subjected to a subsequent sterilization cycle along with a new biological indicator (700), after which, the new biological indicator (700) is incubated and observed for signs of presence or absence of living microorganisms. This process may be repeated until it is determined that the sterilization of the medical device has been effective and the medical device may be used.

When indicator dyes are present during incubation of microorganisms in a liquid growth medium, signs of microorganism metabolism and/or growth may include a color change of the liquid growth medium (for example, as a result of a change in pH in the liquid growth medium). If sterilization has been effective and there are no living microorganisms in the liquid growth medium, there should be no detectable change in the color of the liquid growth medium. If sterilization has not been effective and there are living microorganisms in the liquid growth medium, a detectable color change should occur. In some instances, assessment of color change is made visually, rather than by spectrophotometrically measuring changes in the absorbance spectrum of the liquid growth medium. Thus, when an observer relies solely on visual assessment, the determination of whether or not a sterilization cycle has been successful is subjective and may therefore be susceptible to error.

Another issue associated with reliance upon indicator dyes for signs of microorganism metabolism and/or growth is that it may require a prolonged incubation period in order for a detectable color change in the liquid growth medium to occur. For this reason, methods relying upon color change of indicator dyes may require incubation periods of upwards of 18 to 48 hours, before they can be assessed. Thus, a medical device that has been subjected to a sterilization cycle may be out of use for an extended period of time, particularly if the sterilization cycle is determined to have been ineffective. In some instances, medical instruments may be utilized pending receipt of assessment of the indicator dyes. If, in these instances, the assessment of the indicator dyes is positive for growth, a medical institution may have to notify patients to whom prophylactic antibiotics should then be administered. In any case, if the sterilization cycle is ineffective, then the medical device must not only be subjected to another sterilization cycle, but another biological indicator (700) must then undergo a further incubation period to determine if the subsequent sterilization cycle was effective.

In an effort to accelerate the process of assessing efficacy of a sterilization cycle, and to determine whether or not a medical instrument must undergo another sterilization cycle, some efficacy assessment methods may utilize two indicator reagents. For instance, a first indicator reagent (e.g., one used for fluorescence detection) may be used for microorganism viability detection and a second indicator reagent (e.g., a pH indicator) may be used (as described above) to detect microorganism metabolism and/or growth. In practice, the first indicator may provide a relatively early signal as to whether microorganisms present in the biological indicator (700) are still viable after a sterilization cycle. If the microorganisms are still viable, a decision may be made then to proceed with subjecting the medical instrument to a subsequent sterilization cycle. If, however, the first indicator does not conclusively indicate viability of the microorganisms, then incubation must be continued for the extended period of time that is necessary until the second indicator confirms whether there are signs of microorganism metabolism and/or growth in the biological indicator (700).

In addition to being time consuming, use of more than one indicator reagent as described above may result in interference or conflict in the detection of the one or more indicator reagents in the liquid culture medium. For example, when the second indicator reagent is a pH indicator (e.g., one or more of the pH indicators described below), the pH indicator may conflict or interfere with the fluorescence reading of the first indicator reagent, for example, if the pH indicator emits electromagnetic radiation at a wavelength that is similar to the spectral band of the fluorescence of the first indicator reagent (e.g., such as when the pH indicator exhibits a color shift). Therefore, it may be necessary to include a substrate in the biological indicator (700), wherein the substrate is configured (e.g., formed of an appropriate material) to absorb and/or selectively concentrate the second indicator reagent when positioned in contact with the liquid growth medium to reduce the concentration of the second indicator reagent in the liquid growth medium so that the fluorescence of the first indicator reagent may be accurately assessed.

Examples of pH indicators that may be used include, but are not limited to: thymol blue, tropeolin OO, methyl yellow, methyl orange, bromphenol blue, bromocresol green, methyl red, bromthymol blue, phenol red, neutral red, phenolphthalein, thymolphthalein, alizarin yellow, tropeolin O, nitramine, trinitrobenzoic acid, thymol blue, bromphenol blue, tetrabromphenol blue, bromocresol green, bromocresol purple, bromocresol blue, methyl red, bromthymol blue, phenol red, Congo red, and cresol red. Examples of redox indicators (i.e., oxidation-reduction indicators) that may be used include, but are not limited to, 2,2'-Bipyridine (Ru complex), Nitrophenanthroline (Fe complex), N-Phenylanthranilic acid, 1,10-Phenanthroline (Fe complex), N-Ethoxychrysoidine, 2,2'-Bipyridine (Fe complex), 5,6-Dimethylphenanthroline (Fe complex), o-Dianisidine, Sodium diphenylamine sulfonate, Diphenylbenzidine, Diphenylamine, Viologen, Sodium 2,6-Dibromophenol-indophenol, Sodium 2,6-Dichlorophenol-indophenol, Sodium o-Cresol indophenol, Thionine (syn. Lauth's violet), Methylene blue, Indigotetrasulfonic acid, Indigotrisulfonic acid, Indigodisulfonic acid, Indigomonosulfonic acid, Phenosafranin, Safranin T, and Neutral red.

In some exemplary methods for determining efficacy of a sterilization cycle as disclosed herein, spores that contain a fixed number of copies of a given enzyme in their spore coats when the spores are dormant may be utilized as indicators of the effectiveness of a sterilization cycle. In some embodiments, the enzyme may be more resistant to sterilization conditions than the spores containing it.

In some exemplary methods, an enzyme substrate may be added to the liquid growth medium (732) in order to measure activity of the enzymes. When acted upon by the enzyme, the enzyme-modified substrate forms detectable product(s) that may be detected utilizing any suitable means of protein detection. Exemplary methods of protein detection may be selected from chromogenic measurements, absorption measurements, immunogenic measurements, ligand binding measurements and combinations thereof.

After an effective sterilization cycle, if the spores are no longer viable and the enzymes contained therein have been rendered inactive, then the enzyme substrate will not be modified by the enzyme and no significant change in the concentration of detectable products will occur. However, if after a sterilization cycle, the spores are still viable, then the number of copies of the enzymes in the spores initially remains constant in the liquid growth medium (732) and the modification of the enzyme substrate by the enzyme in the liquid growth medium (732) also remains constant. As a result, the rate of change in the concentration of the detectable products in the liquid growth medium (732) initially increase linearly as a function of time. In the liquid growth medium (732), viable spores will ultimately germinate, grow and multiply exponentially, concurrently producing multiple copies of the enzyme. As copies of the enzyme increase, modification of the enzyme substrate by the enzyme and the concentration of the detectable products in the liquid growth medium increases exponentially as a function of time. If, after a marginal sterilization cycle, the spores are no longer viable, but at least some enzymes have survived, then a linear increase in the concentration of detectable products as a function of time may occur, and no exponential increase in the concentration of the detectable products as a function of time will be observed.

In exemplary methods, monitoring for the presence of detectable products in the liquid growth medium provides an early means of determining whether any spores and/or enzymes survive the sterilization cycle. If no detectable products are present in the liquid growth medium (732), then the spores are no longer viable, the enzymes have been inactivated and the sterilization cycle has been effective. If, however, the concentration of detectable products in the liquid growth medium (732) is observed to increase linearly as a function of time, then early detection of viable spores and/or active enzymes is possible, thereby suggesting that the sterilization cycle was ineffective. Continued monitoring of the concentration of detectable products in the liquid growth medium allows for confirmation that the sterilization cycle was ineffective. For example, if the rate of change in the concentration of detectable products in the liquid growth medium (732) is observed to transition from a linear rate of change to an exponential rate of change, confirmation is provided that the spores in the biological indicator (700) not only remained viable after the sterilization process, but that they are vegetative and multiplying. Advantageously, this determination as to whether a sterilization cycle is effective may be achieved without utilizing any indicator dyes (e.g., pH indicator dyes) that might otherwise interfere with the detection of the detectable products in the liquid growth medium (732).

By monitoring for a shift in the rate of change of the concentration of detectable products, exemplary methods may allow for more rapid confirmation of whether a sterilization cycle has effectively destroyed all of the spores in a biological indicator (700) as compared to methods that require a protracted incubation period for signs of spore metabolism and/or growth to become measurable or quantifiable, particularly due to changes in pH of the liquid growth medium (732). By way of example only, a shift in the rate of change as a function of time of the concentration of detectable products from a linear one to an exponential one may be measured during the incubation period as early as about 5, about 15 minutes, about 30 minutes, about 1 hour, about 2 hours, up to about 12 hours.

Additionally, or alternatively, measuring the rate of change of detectable products may eliminate false positives that result from observing activity of enzymes that have survived a sterilization cycle when the spores that comprise them have not, since without spore growth and multiplication, the number of enzyme copies should not increase. Consequently, liquid growth media (732) of use in the exemplary methods may be substantially free of indicator dyes, "substantially free" as used herein, meaning containing less than 1%, less than 0.5% or 0% by volume of indicator dyes.

In addition, or in the alternative, exemplary methods disclosed herein may utilize one indicator reagent to determine efficacy of a sterilization cycle, rather than two indicator reagents having emission spectra and/or color changes that may potentially conflict or interfere with each other, thus providing for better sensitivity than methods utilizing two indicators. In addition, or in the alternative, utilizing one indicator reagent per the exemplary methods described herein may eliminate the need for including a substrate in the biological indicator (700) that will absorb and/or selectively concentrate one indicator reagent so that it will not interfere with measurement of another indicator. In addition, or in the alternative, exemplary methods may eliminate the use of indicator dyes for signs of microorganism metabolism and/or growth, which may also eliminate subjective assessment of whether a growth medium has undergone color change associated with an ineffective sterilization cycle. In addition, or in the alternative, exemplary methods utilize spores that are known to contain a fixed number of a given enzyme in their coats. Since the enzymes may be more resistant to sterilization than the spores that contain them, the present methods may provide for increased sensitivity in detection of efficacy of a sterilization cycle as compared to methods that detect efficacy by assaying for microorganism metabolism and/or growth.

As mentioned above, the source of biological activity to be utilized in exemplary methods for detecting efficacy of a sterilization cycle may be chosen based upon the resistance of the source to the particular sterilization process to be used in the sterilization cycle. For example, for a steam sterilization process, *Geobacillus stearothermophilus* or spores thereof, can be used. For an ethylene oxide sterilization process, *Bacillus atrophaeus* (formerly *Bacillus subtilis),* or spores thereof, can be used. In some sterilization processes, sterilization process resistant spores can include, but are not limited to, at least one of *Geobacillus stearothermophilus* spores, *Bacillus subtilis* spores, *Bacillus atrophaeus* spores, *Bacillus megaterium* spores, *Bacillus coagulans* spores, *Clostridium sporogenes* spores, *Bacillus pumilus* spores and combinations thereof.

Enzymes and enzyme substrates that may be used to detect efficacy of a sterilization cycle are identified in U.S. Pat. No. 5,073,488, entitled "Rapid Method for Determining Efficacy of a Sterilization Cycle and Rapid Read-Out Biological Indicator," issued December 17, 1991, the disclosure of which is incorporated by reference herein; U.S. Pat. No. 5,418,167, entitled "Rapid Read-Out Biological Indicator," issued May 23, 1995, the disclosure of which is incorporated by reference herein; U.S. Pat. No. 5,223,401, entitled "Rapid Read-Out Sterility Indicator," issued June 29, 1993, the disclosure of which is incorporated by reference herein; and U.S. Pat. No. 9,322,046, entitled "Biological Sterilization Indicator," issued April 26, 2016, the disclosure of which is incorporated by reference herein.

Suitable enzymes may include hydrolytic enzymes and/or enzymes derived from spore-forming microorganisms, such as *Bacillus subtilis.* Enzymes from spore-forming microorganisms that can be useful in exemplary biological indicators may include beta-D-glucosidase, alpha-D-glucosidase, alkaline phosphatase, acid phosphatase, butyrate esterase, caprylate esterase lipase, myristate lipase, leucine aminopeptidase, valine aminopeptidase, chymotrypsin, phosphohydrolase, alpha-D-galactosidase, beta-D-galactosidase, tyrosine aminopeptidase, phenylalanine aminopeptidase, beta-D-glucuronidase, alpha-L-arabinofuranosidase, N-acetyl-beta-glucosaminodase, beta-D-cellobiosidase, alanine aminopeptidase, proline aminopeptidase, fatty acid esterases and combinations thereof.

As noted above, in some exemplary methods for determining efficacy of a sterilization cycle as disclosed herein, enzyme substrates are converted to detectable product. For instance, an enzyme substrate may be characterized by a first emission spectrum (e.g., a first fluorescent emission spectrum) and a detectable product may be characterized by a second emission spectrum (e.g., a second fluorescent emission spectrum).

In some exemplary methods for determining efficacy of a sterilization cycle as disclosed herein, suitable enzyme substrates of use may include fluorogenic enzyme substrates. Useful fluorogenic enzyme substrates may be selected from: fluorogenic 4-methylumbelliferyl derivatives (hydrolysable to 4-methylumbelliferone), derivatives of 7-amido-4-methyl-coumarin (e.g. as disclosed in GB Patent No. 1,547,747 (Nagatsu et al) and European Patent No. 0,000,063 (Sakakibara), each of which is incorporated herein by reference in its entirety), diacetylfluorescein derivatives, fluorescamine and combinations thereof.

Exemplary 4-methylumbelliferyl derivatives may be selected from: 4-methylumbelliferyl-2-acetamido-4,6-O-benzylidene-2-deoxy-β-D-glucopyranoside, 4-methylumbelliferyl acetate, 4-methylumbelliferyl-N-acetyl-β-D-galactosaminide, 4-methylumbelliferyl-N-acetyl-α-D-glucosaminide, 4-methylumbelliferyl-N-acetyl-β-D-glucosaminide, 2'-(4-methylumbelliferyl)-α-D-N-acetyl neuraminic acid, 4-methylumbelliferyl α-L-arabinofuranoside, 4-methylumbelliferyl α-L-arabinoside, 4-methylumbelliferyl butyrate, 4-methylumbelliferyl 13-D-cellobioside, methylumbelliferyl β-D-N,N' diacetyl chitobioside, 4-methylumbelliferyl elaidate, 4-methylumbelliferyl β-D-fucoside, 4-methylumbelliferyl α-L-fucoside, 4-methylumbelliferyl β-L-fucoside, 4-methylumbelliferyl α-D-galactoside, 4-methylumbelliferyl β-D-galactoside, 4-methylumbelliferyl α-D-glucoside, 4-methylumbelliferyl β-D-glucoside, 4-methylumbelliferyl (3-D-glucuronide, 4-methylumbelliferyl p-guanidinobenzoate, 4-methylumbelliferyl heptanoate, 4-methylumbelliferyl α-D-mannopyranoside, 4-methylumbelliferyl β-D-mannopyranoside, 4-methylumbelliferyl oleate, 4-methylumbelliferyl palmitate, 4-methylumbelliferyl phosphate, 4-methylumbelliferyl propionate, 4-methylumbelliferyl stearate, 4-methylumbelliferyl sulfate, 4-methylumbelliferyl β-D-N,N',N"-triacetylchitotriose, 4-methylumbelliferyl 2,3,5-tri-o-benzoyl-α-L-arabinofuranoside, 4-methylumbelliferyl-p-trimethylammonium cinnamate chloride, 4-methylumbelliferyl β-D-xyloside and combinations thereof.

In some exemplary methods in which fluorogenic 4-methylumbelliferyl derivatives that are hydrolysable to 4-methylumbelliferone ("4-MU") are utilized, performing differential absorption spectroscopy to detect the presence of 4-MU may also allow for earlier detection of microorganism metabolism and/or growth.

One means of detecting 4-MU resulting from enzyme cleavage of the alpha-4MU bond, is to excite 4-MU with light having a wavelength of 347 nanometers ("nm"), while performing fluorimetric measurements in its blue fluorescence band at 445 nm. However, this is a rate-limiting step in that limits the speed with which microorganism metabolism and/or growth may be measured. Thus, in some exemplary embodiments, methods for detecting efficacy of a sterilization cycle comprise taking a baseline fluorimetric measurement of the alpha-4MU bond before it is cleaved and then compare it to the fluorimetric measurement when exciting 4-MU with light having a wavelength of 347 nanometers ("nm"), thus eliminating the need to perform fluorimetric measurements in the blue fluorescence band of 4-MU at 445 nm. Without wishing to be bound by theory, it is believed that performing differential absorption spectroscopy to detect the early presence of 4-MU is possible, because the fluorescence intensity of 4-MU is pH dependent, i.e., 4-MU is colorless at pH of a 7.0 and exhibits blue fluorescence at pH 7.5, the blue fluorescence increasing up to, and plateauing at, pH 10.7. Since the fluorescence of 4-MU at a pH of 10.3 is approximately 100 times as intense as it is at pH 7.4, the formation of 4-MU in the liquid growth medium can be readily monitored by differential absorption spectroscopy at 347 nm or by fluorimetric measurements taken at a pH of 10.8. It should be understood that "at a pH" as used herein means "in a band having a center frequency of."

Exemplary 7-amido-4-methylcoumarin derivatives may be selected from: L-alanine-7-amido-4-methylcoumarin, L-proline 7-amido-4-methylcoumarin, L-tyrosine-7-amido-4-methylcoumarin, L-leucine-7-amido-4-methylcoumarin, L-phenylalanine-7-amido-4-methylcoumarin, 7-glutarylphenylalanine-7-amido-4-methylcoumarin and combinations thereof.

Exemplary peptide derivatives of 7-amido-4-methyl coumarin may be selected from: N-t-BOC-11e-Glu-Gly-Arg 7-amido-4-methylcoumarin, N-t-BOC-Leu-Ser-Thr-Arg 7-amido-4-methylcoumarin, N-CBZ-Phe-Arg 7-amido-4-methyl-coumarin, Pro-Phe-Arg 7-amido-4-methylcoumarin, N-t-BOC-Val-Pro-Arg 7-amido-4-methylcoumarin, N-glutaryl-Gly-Arg 7-amido-4-methylcoumarin and combinations thereof.

Exemplary diacetylfluorescein derivatives may be selected from: fluorescein diacetate, fluorescein di-(β-D-galactopyranoside), fluorescein dilaurate and combinations thereof.

In exemplary methods in which the biological activity to be detected is alpha-D-glucosidase, chymotrypsin, or fatty acid esterase, e.g., from *Geobacillus stearothermophilus,* exemplary fluorogenic enzyme substrates may be selected from: 4-methylumbelliferyl-alpha-D-glucoside, 7-glutarylphenylalanine-7-amido-4-methyl coumarin, 4-methylumbelliferyl heptanoate and combinations thereof. In exemplary methods in which the biological activity to be detected is alpha-L-arabinofuranosidase, e.g., derived from *Bacillus subtilis,* the fluorogenic enzyme substrate may be 4-methylumbelliferyl-alpha-L-arabinofuranoside. In exemplary methods in which the biological activity to be detected is beta-D-glucosidase, e.g., derived from *Bacillus subtilis,* the fluorogenic enzyme substrate may be 4-methylumbelliferyl-beta-D-glucoside.

In order to detect a biological activity comprising an enzyme, it may be important to choose the appropriate enzyme activity to be detected and the enzyme substrates that will react with the enzyme so as to produce a product which can be detected either by its fluorescence, color, etc. (see M. Roth, Methods of Biochemical Analysis, Vol. 7, D. Glock, Ed., Interscience Publishers, New York, N.Y., 1969, which is incorporated herein by reference in its entirety). The appropriate enzyme substrate to be utilized may depend upon the biological activity to be detected.

As discussed above, FIG. 5 shows an exemplary set of steps that may be used to initiate biological indicator (700) analysis cycle by biological indicator analyzer (800). During the incubation period (block 914), or alternatively, after a predetermined incubation period, light source (830) associated with the selected well (810) containing biological indicator (700) is activated and sensor (840) monitors responsive fluorescence of fluid (732) in biological indicator (700). In some exemplary embodiments, the light source (830) associated with the selected well (810) containing biological indicator (700) is activated prior to the incubation period to obtain a baseline reading.

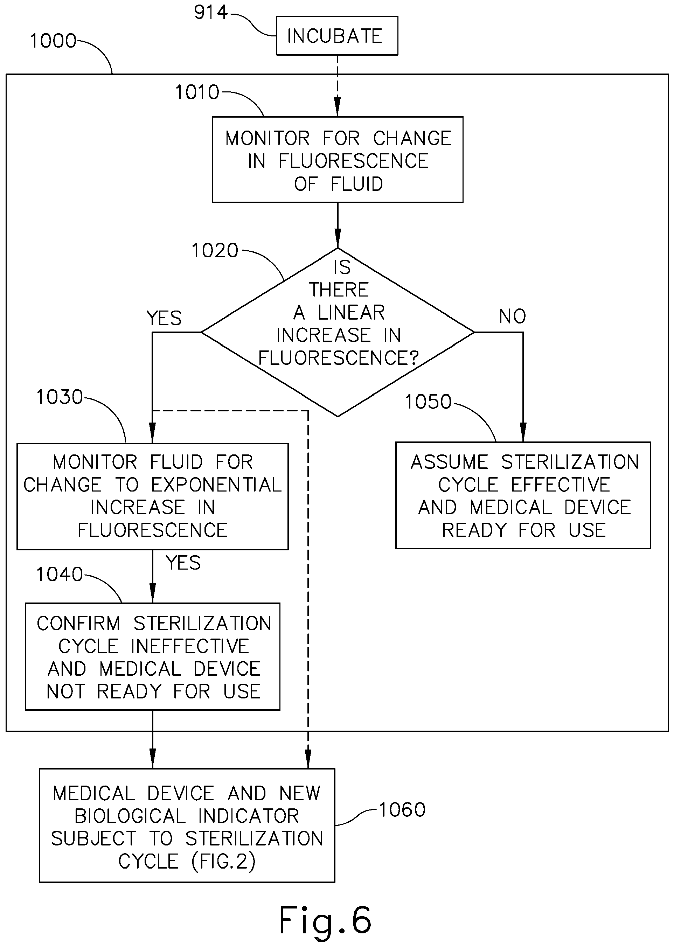

FIG. 6 shows an exemplary method (1000) for detecting efficacy of a sterilization cycle using a biological indicator (700) that has been subject to an incubation period (block 914) as shown and described in relation to FIG. 5. In the present example, fluid (732) in biological indicator (700) contains spores that each contain a fixed number of copies of an enzyme and an enzyme substrate characterized by a first fluorescence emission spectrum.

Either after a given incubation period, or during incubation, (block 914) fluorescence of fluid (732) is monitored (block 1010), for example, in a selected well (810) of a biological analyzer (800). A query (block 1020) of whether there is a linear increase in the fluorescence of the fluid (732) as a function of time is undertaken. If there is no linear increase in the fluorescence of the fluid as a function of time, then it is assumed that the sterilization cycle was effective and the medical device is ready for use (block 1050). If there is a linear increase in fluorescence of the fluid as a function of time, then it is assumed that the sterilization cycle was not effective, i.e., the spores and/or the enzymes are still viable. At this point, it may be assumed that the sterilization cycle was ineffective and the medical device and another biological indicator (700) may then be subject to a subsequent sterilization cycle per the exemplary steps shown in FIG. 2 (block 1060). The steps performed by indicator analyzer (800) per FIG. 5 may then be repeated. Alternatively, the fluorescence of the fluid may continue to be monitored for detection of a change in the rate of increase of the fluorescence of the fluid as a function of time (block 1030). If the rate of change in fluorescence of the fluid as a function of time changes from a linear rate to an exponential rate, then it is confirmed that the spores are vegetative and that the sterilization cycle was ineffective (block 1040). The medical device and another biological indicator (700) may then be subject to a subsequent sterilization per the exemplary steps shown in FIG. 2 (block 1060). The steps performed by indicator analyzer (800) per FIG. 5 may then be repeated. Biological indicator is then subject to monitoring (block 1010) and query (block 1020) until a biological indicator (700) that is subject to a sterilization cycle with the medical device, is incubated and shows no increase in fluorescence of the fluid as a function of time and the medical device is ready for use (block 1050).

### VII. Exemplary Combinations

The following examples relate to various non-exhaustive ways in which the teachings herein may be combined or applied. It should be understood that the following examples are not intended to restrict the coverage of any claims that may be presented at any time in this application or in subsequent filings of this application. No disclaimer is intended. The following examples are being provided for nothing more than merely illustrative purposes. It is contemplated that the various teachings herein may be arranged and applied in numerous other ways. It is also contemplated that some variations may omit certain features referred to in the below examples. Therefore, none of the aspects or features referred to below should be deemed critical unless otherwise explicitly indicated as such at a later date by the inventors or by a successor in interest to the inventors. If any claims are presented in this application or in subsequent filings related to this application that include additional features beyond those referred to below, those additional features shall not be presumed to have been added for any reason relating to patentability.

### Example 1

A method of detecting biological activity in a biological indicator, comprising:
(a) providing a known quantity of spores containing a fixed number of copies of an enzyme;
(b) providing a liquid growth medium comprising substrates of the enzyme, wherein the substrates have a first emission spectrum, and wherein the substrates are configured to be converted by the enzyme to substrate derivatives having a second emission spectrum;
(c) exposing the spores to the liquid growth medium; (d) measuring the liquid growth medium for the second emission spectrum; and (e) detecting as a function of time either:
   (i) no change in the second emission spectrum; or (ii) a linear increase in the second emission spectrum.

### Example 2

The method of example 1, wherein a linear increase in the second emission spectrum is detected, the method further comprising monitoring the second emission spectrum for a change from a linear increase in the second emission spectrum to an exponential increase in the second emission spectrum.

### Example 3

The method of example 1, wherein the spores are selected from *Geobacillus stearothermophilus* spores, *Bacillus subtilis* spores, *Bacillus atrophaeus* spores, *Bacillus megaterium* spores, *Bacillus coagulans* spores, *Clostridium sporogenes* spores, *Bacillus pumilus* spores and combinations thereof.

### Example 4

The method of example 3, wherein the enzymes are selected from beta-D-glucosidase, alpha-D-glucosidase, alkaline phosphatase, acid phosphatase, butyrate esterase, caprylate esterase lipase, myristate lipase, leucine aminopeptidase, valine aminopeptidase, chymotrypsin, phosphohydrolase, alpha-D-galactosidase, beta-D-galactosidase, tyrosine aminopeptidase, phenylalanine aminopeptidase, beta-D-glucuronidase, alpha-L-arabinofuranosidase, N-acetyl-beta-glucosaminodase, beta-D-cellobiosidase, alanine aminopeptidase, proline aminopeptidase, fatty acid esterases and combinations thereof.

### Example 5

The method of example 1, wherein the liquid growth media is substantially free of indicator dye.

### Example 6

The method of example 2, wherein the change from a linear increase in the second emission spectrum to an exponential increase in the second emission spectrum occurs in less than about 2 hours.

### Example 7

The method of example 2, wherein the change from a linear increase in the second emission spectrum to an exponential increase in the second emission spectrum occurs in less than about 1 hour.

### Example 8

The method of example 2, wherein the change from a linear increase in the second emission spectrum to an exponential increase in the second emission spectrum occurs in less than about 30 minutes.

### Example 9

A method of using a biological indicator, wherein the biological indicator comprises: (i) a frangible ampoule containing a first liquid, the first liquid comprising liquid growth medium and a fluorogenic enzyme substrate having a first emission spectrum, wherein the fluorogenic enzyme substrate is configured to be converted by the enzyme to a fluorescent derivative with a second emission spectrum, and (ii) a carrier supporting a known quantity of bacterial spores containing a fixed number of copies of an enzyme, the method comprising: (a) fracturing the frangible ampule; (b) exposing the liquid growth medium to the bacterial spores to form a second liquid; (c) incubating the second liquid; and (d) monitoring the second liquid and detecting as a function of time either: (i) no change in the second emission spectrum, or (ii) a linear increase in the second emission spectrum.

### Example 10

The method of example 9, wherein a linear increase in the second emission spectrum is detected, the method further comprising monitoring the second emission spectrum for a change from a linear increase in the second emission spectrum to an exponential increase in the second emission spectrum.

### Example 11

The method of example 9, wherein the first liquid and second liquid are substantially free of indicator dye.

### Example 12

The method of example 9, wherein the first liquid and the second liquid are colorless.

### Example 13

The method of example 10, wherein the change from a linear increase in the second emission spectrum to an exponential increase in the second emission spectrum occurs in less than about 2 hours.

### Example 14

The method of example 10, wherein the change from a linear increase in the second emission spectrum to an exponential increase in the second emission spectrum occurs in less than about 1 hour.

### Example 15

The method of example 10, wherein the change from a linear increase in the second emission spectrum to an exponential increase in the second emission spectrum occurs in less than about 30 minutes.

### Example 16

The method of example 10, wherein the change from a linear increase in the second emission spectrum to an exponential increase in the second emission spectrum occurs in less than about 5 minutes.

### Example 17

The method of example 9, wherein the biological indicator is free of a substrate that is configured to absorb and/or selectively concentrate an indicator reagent.

### Example 18

The method of example 9, wherein the spores are selected from *Geobacillus stearothermophilus* spores, *Bacillus subtilis* spores, *Bacillus atrophaeus* spores, *Bacillus megaterium* spores, *Bacillus coagulans* spores, *Clostridium sporogenes* spores, *Bacillus pumilus* spores and combinations thereof.

### Example 19

The method of example 9, wherein the enzymes are selected from beta-D-glucosidase, alpha-D-glucosidase, alkaline phosphatase, acid phosphatase, butyrate esterase, caprylate esterase lipase, myristate lipase, leucine aminopeptidase, valine aminopeptidase, chymotrypsin, phosphohydrolase, alpha-D-galactosidase, beta-D-galactosidase, tyrosine aminopeptidase, phenylalanine aminopeptidase, beta-D-glucuronidase, alpha-L-arabinofuranosidase, N-acetyl-beta-glucosaminodase, beta-D-cellobiosidase, alanine aminopeptidase, proline aminopeptidase, fatty acid esterases and combinations thereof.

### Example 20

A method of confirming efficacy of sterilization of spores, the method comprising: (a) providing a biological indicator comprising *Geobacillus stearothermophilus* spores having spore coats, wherein the spore coats contain alphaglucosidase; (b) providinga liquid growth medium that is substantially free of indicator dye, the liquid growth medium comprising 4-methylumbelliferyl-α-D-glucoside, wherein the 4-methylumbelliferyl-α-D-glucoside has a first emission spectrum, and wherein the 4-methylumbelliferyl-α-D-glucoside is converted by the alphaglucosidase to a fluorescent derivative with a second emission spectrum; (c) exposing the liquid growth medium to the *Geobacillus stearothermophilus* spores; and (d) detecting as a function of time a change from a linear increase in the second emission spectrum to an exponential increase in the second emission spectrum.

### VIII. Miscellaneous

It should be appreciated that any patent, publication, or other disclosure material, in whole or in part, that is said to be incorporated by reference herein is incorporated herein only to the extent that the incorporated material does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

Having shown and described various embodiments of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, embodiments, geometries, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. A method of detecting biological activity in a biological indicator, comprising:
(a) providing a known quantity of spores containing a fixed number of copies of an enzyme;
(b) providing a liquid growth medium comprising substrates of the enzyme, wherein the substrates have a first emission spectrum, and wherein the substrates are configured to be converted by the enzyme to substrate derivatives having a second emission spectrum;
(c) exposing the spores to the liquid growth medium;
(d) measuring the liquid growth medium for the second emission spectrum; and
(e) detecting as a function of time either:
(i) no change in the second emission spectrum; or
(ii) a linear increase in the second emission spectrum.

2. The method of claim 1, wherein a linear increase in the second emission spectrum is detected, the method further comprising monitoring the second emission spectrum for a change from a linear increase in the second emission spectrum to an exponential increase in the second emission spectrum.

3. The method of claim 1 or claim 2, wherein the spores are selected from *Geobacillus stearothermophilus* spores, *Bacillus subtilis* spores, *Bacillus atrophaeus* spores, *Bacillus megaterium* spores, *Bacillus coagulans* spores, *Clostridium sporogenes* spores, *Bacillus pumilus* spores and combinations thereof.

4. The method of claim 3, wherein the enzymes are selected from beta-D-glucosidase, alpha-D-glucosidase, alkaline phosphatase, acid phosphatase, butyrate esterase, caprylate esterase lipase, myristate lipase, leucine aminopeptidase, valine aminopeptidase, chymotrypsin, phosphohydrolase, alpha-D-galactosidase, beta-D-galactosidase, tyrosine aminopeptidase, phenylalanine aminopeptidase, beta-D-glucuronidase, alpha-L-arabinofuranosidase, N-acetyl-beta-glucosaminodase, beta-D-cellobiosidase, alanine aminopeptidase, proline aminopeptidase, fatty acid esterases and combinations thereof.

5. The method of any one of the preceding claims, wherein the liquid growth media is substantially free of indicator dye.

6. The method of any one of claims 2 to 5, wherein the change from a linear increase in the second emission spectrum to an exponential increase in the second emission spectrum occurs in less than about 2 hours.

7. The method of any one of claims 2 to 6, wherein the change from a linear increase in the second emission spectrum to an exponential increase in the second emission spectrum occurs in less than about 1 hour.

8. The method of any one of claims 2 to 7, wherein the change from a linear increase in the second emission spectrum to an exponential increase in the second emission spectrum occurs in less than about 30 minutes.

9. A method of using a biological indicator, wherein the biological indicator comprises:
(i) a frangible ampoule containing a first liquid, the first liquid comprising liquid growth medium and a fluorogenic enzyme substrate having a first emission spectrum, wherein the fluorogenic enzyme substrate is configured to be converted by the enzyme to a fluorescent derivative with a second emission spectrum, and
(ii) a carrier supporting a known quantity of bacterial spores containing a fixed number of copies of an enzyme,
the method comprising:
(a) fracturing the frangible ampule;
(b) exposing the liquid growth medium to the bacterial spores to form a second liquid;
(c) incubating the second liquid; and
(d) monitoring the second liquid and detecting as a function of time either:
(i) no change in the second emission spectrum, or
(ii) a linear increase in the second emission spectrum.

10. The method of claim 9, wherein a linear increase in the second emission spectrum is detected, the method further comprising monitoring the second emission spectrum for a change from a linear increase in the second emission spectrum to an exponential increase in the second emission spectrum.

11. The method of claim 9 or claim 10, wherein the first liquid and second liquid are substantially free of indicator dye.

12. The method of any one of claims 9 to 11, wherein the first liquid and the second liquid are colorless.

13. The method of any one of claims 10 to 12, wherein the change from a linear increase in the second emission spectrum to an exponential increase in the second emission spectrum occurs in less than about 2 hours, or
wherein the change from a linear increase in the second emission spectrum to an exponential increase in the second emission spectrum occurs in less than about 1 hour, or
wherein the change from a linear increase in the second emission spectrum to an exponential increase in the second emission spectrum occurs in less than about 30 minutes, or
wherein the change from a linear increase in the second emission spectrum to an exponential increase in the second emission spectrum occurs in less than about 5 minutes.

14. The method of any one of claims 9 to 13, wherein the biological indicator is free of a substrate that is configured to absorb and/or selectively concentrate an indicator reagent.

15. The method of any one of claims 9 to 14, wherein the spores are selected from *Geobacillus stearothermophilus* spores, *Bacillus subtilis* spores, *Bacillus atrophaeus* spores, *Bacillus megaterium* spores, *Bacillus coagulans* spores, *Clostridium sporogenes* spores, *Bacillus pumilus* spores and combinations thereof.

16. The method of any one of claims 9 to 15, wherein the enzymes are selected from beta-D-glucosidase, alpha-D-glucosidase, alkaline phosphatase, acid phosphatase, butyrate esterase, caprylate esterase lipase, myristate lipase, leucine aminopeptidase, valine aminopeptidase, chymotrypsin, phosphohydrolase, alpha-D-galactosidase, beta-D-galactosidase, tyrosine aminopeptidase, phenylalanine aminopeptidase, beta-D-glucuronidase, alpha-L-arabinofuranosidase, N-acetyl-beta-glucosaminodase, beta-D-cellobiosidase, alanine aminopeptidase, proline aminopeptidase, fatty acid esterases and combinations thereof.

17. A method of confirming efficacy of sterilization of spores, the method comprising:
(a) providing a biological indicator comprising Geobacillus *stearothermophilus* spores having spore coats, wherein the spore coats contain alphaglucosidase;
(b) providing a liquid growth medium that is substantially free of indicator dye, the liquid growth medium comprising 4-methylumbelliferyl-α-D-glucoside, wherein the 4-methylumbelliferyl-α-D-glucoside has a first emission spectrum, and wherein the 4-methylumbelliferyl-α-D-glucoside is converted by the alphaglucosidase to a fluorescent derivative with a second emission spectrum;
(c) exposing the liquid growth medium to the Geobacillus *stearothermophilus* spores; and
(d) detecting as a function of time a change from a linear increase in the second emission spectrum to an exponential increase in the second emission spectrum.
